# EUROPEAN PATENT SPECIFICATION

(11) **EP 0 297 580 B1**
(45) Date of publication and mention of the grant of the patent: **20.01.1999**
(21) Application number: 88110480.6
(22) Date of filing: 30.06.1988
(51) Int. Cl.: C07D 417/12, A61K 31/425

(54) **Amorphous form of aztreonam**
Amorphe Form von Aztreonam
Forme amorphe de l'aztréoname

(30) Priority: 01.07.1987 US 68392
(43) Date of publication of application: 04.01.1989
(73) Proprietor: E.R. SQUIBB & SONS, INC., Princeton, New Jersey 08543-4000 (US)
(72) Inventor: Varia, Sailesh Amilal, Plainsboro New Jersey (US); Reier, George Eugene, Somerset New Jersey (US); Pipkin, James Douglas, Lawrence Kansas (US)
(74) Representative: VOSSIUS & PARTNER

(56) References cited:
- EP-A- 0 070 024
- EP-A- 0 107 276
- CHEMICAL ABSTRACTS, vol. 106, no. 22, 1st June 1987, abstract no. 182496d, Columbus, Ohio, US; UNITED STATES FOOD AND DRUG ADMINISTRATION: "Antibiotic drugs; aztreonam for injection", & FED. REGIST. 13th Feb. 1987, 52(30), 4610-16

## Description

The present invention relates to a new non-crystalline amorphous form of [3S-[3α(Z),4β]]-3-[[2-amino-4-thiazolyl[1-carboxy-l-methylethoxy) imino]acetyl]amino]-4-methyl-2-oxo-1-azetidine-sulfonic acid and its pharmaceutically acceptable salts which will be referred to as the amorphous form of aztreonam. This amorphous form of aztreonam is prepared by freeze-drying methods. The use of amorphous aztreonam in pharmaceutical formulations results in a product with good stability along with low particulate contamination.

In accordance with the present invention, a new non-crystalline amorphous form of [3S-[3α(Z),4β]]-3-[[2-amino-4-thiazolyl[1-carboxy-1-methylethoxy)imino]acetyl]amino]-4-methyl-2-oxo-1-azetidinesulfonic acid and its pharmaceutically acceptable salts are provided. This amorphous form of aztreonam is prepared by freeze drying the alpha or beta forms of aztreonam. A mixture of both forms can also be used. This freeze-dried form of aztreonam is useful in the formulation of aztreonam for injection. As mentioned above, the preferred embodiment of this invention is in the use of the alpha form of aztreonam. The preparation of this freeze-dried dosage form of aztreonam allows for the use of non-sterile starting materials. These non-sterile materials are later made sterile by a filtering process.

The freeze-dried or lyophilized L-arginine aztreonam salt for injection is prepared by mixing the required amount of alpha or beta aztreonam and 90% of the required L-arginine together. For a 1 gram sample of aztreonam the required amount of L-arginine is 0.7 to 0.84 grams to bring the pH of the solution to about five. Alternatively, based on in-process titration, 100% of the L-arginine required for preparation is used to bring the pH of the solution to about 5.0. This solution is prepared by dissolving the dry mixture of aztreonam and L-arginine in about 80% of the required amount of water for injection. After the pH has been adjusted to 5.0 with more L-arginine, if required, the solution is brought to final volume with water. The solution is clarified and aseptically filtered. This solution is then transferred into an appropriate container. The fill volumes of the bulk solution will vary with the concentration of aztreonam in solution and the potency per container that is required. The solution is then freeze-dried by conventional methods.

The lyophilized product is administered by reconstituting the product with 3 ml of diluent per gram of aztreonam for intramuscular use, 10 ml. per gram of aztreonam for intravenous bolus use and 50 to 100 ml. per gram for intravenous infusion use. Acceptable diluents are water, and others known to one skilled in the art. The injectable product has an aztreonam concentration of about 250 mg/ml for intramuscular, 90 mg/ml for intravenous bolus and 10 to 20 mg/ml for intravenous infusion use.

The bulk solution concentration prior to freeze drying of aztreonam can be varied. Instead of preparing a bulk solution containing 1 g aztreonam per 10 ml of solution, solutions ranging from 1 g in 6 ml to 14 ml of water can be prepared. The preferred concentration of the bulk solution is 11% weight/volume of aztreonam. However, the potency per container can range from 0.5 g to 2 g and this is obtained by adjusting the fill volume of the solution prior to filling. The amount of L-arginine will vary according to the following table.

**Table 1**

| 0.5 g Potency | |
|---|---|
| Ingredient | Per Container |
| Aztreonam | 0.5 g |
| L-Arginine | 0.35 - 0.42 g |

| 1 g Potency | |
|---|---|
| Ingredient | Per Container |
| Aztreonam | 1.0 g |
| L-Arginine | 0.7 - 0.84 g |

| 2 g Potency | |
|---|---|
| Ingredient | Per Container |
| Aztreonam | 2.0 g |
| L-Arginine | 1.4 - 1.68 g |

The freeze-dried formulation of aztreonam utilizes the previously known alpha and beta forms of aztreonam. This freeze-dried amorphous form of aztreonam results in good product stability, less particulate contamination, less variation in pH and potency from batch to batch, sterility of the raw material is not required and the lyophilized product dissolves quicker in water than the previously known forms of aztreonam.

It should be noted that other basic materials can be mixed with crystalline aztreonam to yield the desired lyophilized aztreonam salt product for reconstitution. Among these are salts, prepared using sodium carbonate, sodium bicarbonate, sodium citrate, sodium phosphate and sodium hydroxide. The following examples illustrate the preparation of the amorphous antibiotically active aztreonam salts of this invention.

### Example 1

To 5.04 kg of beta aztreonam activity was added 3.45 kg of L-arginine and the two powders were mixed. The powder blend was added to 40 kg of water for injection with vigorous agitation until the powders were dissolved. The pH of the solution was adjusted to 5.0 with an additional 442 grams of L-arginine to the solution. The final batch volume was adjusted to 55 liters with addition of more water for injection. The solution was filtered through a clarification filter and a 0.2 micron sterile filter into a sterile tank. The following aliquots of the solution were filled into vials to provide the following potencies:

| Fill Volume | Aztreonam Potency/Vial |
|---|---|
| 6 ml | 0.55 g |
| 12 ml | 1.1 g |
| 24 ml | 2.2 g |

A fluted stopper partially open was placed on the vial and the contents lyophilized using standard methods. Upon lyophilization the vials were stoppered under partial vacuum and then sealed. The resultant product is a white to slight yellow cake or fragmented cake.

### Example 2

To 12.1 kg of alpha aztreonam activity was added 8.3 kg of L-arginine and the two powders were mixed. To the powder blend was added 85.0 kg of water for injection with vigorous agitation and mixed until the powders were dissolved. The pH of the solution was adjusted to 5.0 with addition of 819.7 grams of L-arginine to the solution. The final batch volume was adjusted to 110 liters with addition of more water for injection. The solution was filtered through a clarification filter and a 0.2 micron sterile filter into a sterile tank. The following aliquots of the solution were filled into vials to provide the following potencies:

| Fill Volume | Aztreonam Potency/Vial |
|---|---|
| 5 ml | 0.55 g |
| 10 ml | 1.1 g |
| 20 ml | 2.2 g |

A fluted stopper partially open was placed on the vial and the contents lyophilized using standard methods.

### Example 3

An in-process titration on a sample of the batch of alpha aztreonam and L-arginine to be used for the preparation was performed. From the titration, the amount of L-arginine required per gram of aztreonam activity to obtain a pH of 5.0 in solution was determined to be .772 grams of L-arginine.

To 4.4 kg of alpha aztreonam activity, 3.4 kgs of L-arginine was added and mixed. The blend, with vigorous mixing was added to a sufficient quantity of water for injection to make a final batch size of 40 liters. The solution was filtered through a clarification filter and a 0.2 micron sterile filter into a sterile tank. The following aliquots of the solution were placed into vials to provide the following potencies:

| Fill Volume | Aztreonam Potency/Vial |
|---|---|
| 5 ml | 0.55 g |
| 10 ml | 1.1 g |
| 20 ml | 2.2 g |

A fluted stopper was placed partially open on vial and the contents lyophilized using standard methods.

### Example 4

An in-process titration on a sample of the batch of alpha aztreonam and L-arginine to be used for the preparation was performed. From the titration, the amount of L-arginine required per gram of aztreonam activity to obtain a pH of 5.0 in solution was determined to be .765 grams of L-arginine. 1.69 kg of alpha aztreonam activity was added to 1.29 kg of L-arginine. The blend was mixed with sufficient quantity of water for injection to make a final batch size of 9.66 liters. The solution was filtered through a clarification filter and a 0.2 micron sterile filter into a sterile tank. The following aliquots of the solution were placed into vials to provide the following potencies:

| Fill Volume | Aztreonam Potency/Vial |
|---|---|
| 3.15 ml | 0.55 g |
| 6.30 ml | 1.1 g |
| 12.60 ml | 2.2 g |

A fluted stopper was placed partially open on a vial and the contents lyophilized using standard methods.

## Claims

1. The non-crystalline, amorphous form of [3S-[3α(Z), 4β]]-3[[2-amino-4-thiazolyl[1-carboxy-1-methylethoxy)imino]acetyl]amino]-4-methyl-2-oxo-1-azetidinesulfonic acid and its pharmaceutically acceptable salts.

2. The compound of claim 1 wherein the pharmaceutically acceptable salt is the L-arginine salt.

3. A method of preparing the compound of claims 1 and 2 which comprises mixing crystalline aztreonam with L-arginine, dissolving the mixture in water and freeze-drying the dissolved mixture.

4. A pharmaceutical preparation comprising a compound according to claim 1 or its pharmaceutically acceptable salts.

5. A pharmaceutical preparation according to claim 2 wherein the pharmaceutically acceptable salt is the L-arginine salt.

6. A pharmaceutical preparation according to claims 4 and 5 having antibiotic activity.

## Patentansprüche

1. Nicht-kristalline, amorphe Form der [3S-[3α(Z), 4β]]-3[[2-Amino-4-thiazolyl[1-carboxy-1-methylethoxy)imino]acetyl]amino]4-methyl-2-oxo-1-azetidinsulfonsäure und ihre pharmazeutisch verträglichen Salze.

2. Verbindung nach Anspruch 1, wobei das pharmazeutisch verträgliche Salz das L-Arginin-Salz ist.

3. Verfahren zur Herstellung der Verbindung nach Anspruch 1 und 2, welches das Mischen von kristallinem Aztreonam mit L-Arginin, das Auflösen des Gemisches in Wasser und das Gefriertrocknen des aufgelösten Gemisches umfaßt.

4. Arzneimittel, umfassend eine Verbindung nach Anspruch 1 oder seine pharmazeutisch verträglichen Salze.

5. Arzneimittel nach Anspruch 2, wobei das pharmazeutisch verträgliche Salz das L-Arginin-Salz ist.

6. Arzneimittel nach Anspruch 4 und 5, das antibiotische Aktivität hat.

## Revendications

1. Forme non cristalline, amorphe de l'acide [3S-[3α(Z),4β]]-3[[2-amino-4-thiazolyl[1-carboxy-1-méthyléthoxy) imino]acétyl]amino]-4-méthyl-2-oxo-1-azétidine sulfonique et ses sels pharmaceutiquement acceptables.

2. Composé de la revendication 1 où le sel pharmaceutiquement acceptable est le sel de la L-arginine.

3. Méthode de préparation du composé des revendications 1 et 2 qui comprend le mélange d'aztréonam cristallin avec de la L-arginine, la dissolution du mélange dans l'eau et la lyophilisation du mélange dissous.

4. Préparation pharmaceutique comprenant un composé selon la revendication 1 ou ses sels pharmaceutiquement acceptables.

5. Préparation pharmaceutique selon la revendication 2 où le sel pharmaceutiquement acceptable est le sel de la L-arginine.

6. Préparation pharmaceutique selon les revendications 4 et 5 ayant une activité antibiotique.
